# NEUE EUROPÄISCHE PATENTSCHRIFT

(11) **EP 2 004 149 B2**
(45) Veröffentlichungstag und Bekanntmachung des Hinweises auf die Entscheidung über den Einspruch: **04.03.2015**
(45) Hinweis auf die Patenterteilung: 21.12.2011
(21) Anmeldenummer: 07724218.8
(22) Anmeldetag: 12.04.2007
(51) Int. Cl.: A61K 9/26, A61P 29/02

(54) **LEFLUNOMID ENTHALTENDE PHARMAZEUTISCHE ZUSAMMENSETZUNGEN**
PHARMACEUTICAL COMPOSITIONS CONTAINING LEFLUNOMID
COMPOSITIONS PHARMACEUTIQUES CONTENANT DU LÉFLUNOMIDE

(30) Priorität: 13.04.2006 DE 102006017896
(43) Veröffentlichungstag der Anmeldung: 24.12.2008
(73) Patentinhaber: Alfred E. Tiefenbacher (GmbH & Co. KG), 22767 Hamburg (DE)
(72) Erfinder: Dr. RUCHATZ, Dieter, 22767 Hamburg (DE); Dr. PETERS, Harm, 22869 Schenefeld (DE)
(74) Vertreter: Feldmann, Ute
(86) Internationale Anmeldenummer: PCT/EP2007/003277
(87) Internationale Veröffentlichungsnummer: WO 2007/118684

(56) Entgegenhaltungen:
- EP-A- 0 797 989
- EP-A1- 1 920 772
- EP-A2- 0 413 329
- WO-A-96/33745
- WO-A1-96/24338
- US-A- 5 677 335
- US-A1- 2006 024 376
- P. SOMASUNDARAN: "Encyclopedia of Surgace and Colloid Science", 2006, TAYLOR&FRANCIS, NEW YORK, LONDON * page 4541, right-hand column, last paragraph - page 4542, left-hand column, paragraph 1 *
- T. EICHER ET AL.: 'Chemie der Heterozyklen (Struktur, Reaktionen und Synthesen)', 1994, GEORG THIEME VERLAG, STUTTGART - NEW YORK Seiten 138 - 139
- H. DELONCA ET AL.: 'Granulation de l'acide acetylsalicylique par voie humide, Influence du pH et de certains adjuvants de stabilisation' FARMACO PRAT. Bd. 30, Nr. 2, Februar 1975, Seiten 89 - 97
- L. J. EDWARDS: 'A determination of the thermodynamic dissociation constant and a study of the reaction kinetics by ultra-violet spectrophotometry' THE HYDROLYSIS OF ASPIRIN Bd. 46, 1950, Seiten 723 - 735
- K. H. BAUER ET AL.: 'Pharmazeutische Technologie', Bd. 5, 1997, GUSTAV FISCHER, STUTTGART Seiten 232FF - 313FF
- A. S. KALGUTKAR ET AL.: 'In vitro metabolism studies on the isoxazole ring scission in the anti-inflammatory agent leflunomide to its active a-cyanoenol metabolite A771726: mechanistic similarities with the cytchrome P450-catalysed dehydrogenation of aldoximes' DRUG METABOLISM AND DISPOSITION Bd. 31, Nr. 10, 2003, Seiten 1240FF - 1243
- Center for Drug Evaluation and Research, Pharmacology Reviews, NDA 20-905, Seite 3, VÖ:2. Oktober 2003
- US Pharmacopeia, USP 28, NF 23, Seite 9
- W. J. LYMAN, PH.D. ET AL.: 'Handbook of chemical property estimation methods environmental behavior of organic compounds', 1990, AMERICAN CHEMICAL SOCIETY, WHASHINGTON, DC Seite 7-2
- AUTERHOFF ET AL.: 'Lehrbuch der Pharmazeutischen Chemie', Bd. 13, 1994, WISSENSCHAFTLICHE VERLAGSGESELLSCHAFT MBH, STUTTGART Seite 447

## Beschreibung

Die Erfindung betrifft Leflunomid enthaltende pharmazeutische Zusammensetzungen mit verbesserter Lagerstabilität.

Der pharmazeutische Wirkstoff Leflunomid (**I**) mit der chemischen Bezeichnung 5-Methyl-*N*-[4-(trifluormethyl)-phenyl]-4-isoxazolcarboxamid ist ein Antirheumatikum aus der Gruppe der Isoxazole und wird als Basistherapeutikum eingesetzt.

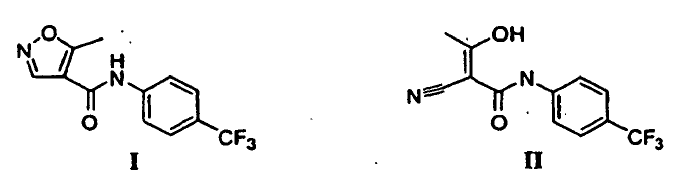

Leflunomid ist ein Prodrug und besitzt immunmodulierende Eigenschaften. Der aktive Metabolit des Leflunomids (Teriflunomid, **II**) entsteht durch Öffnung des Isoxazolrings zu einem Malonitrilamid. *In vitro* hemmt dieses Malonitrilamid die Vermehrung der T-Lymphozyten und die T-zellabhängige Auto-Antikörperbildung in B-Lymphozyten. Seine immunmodulierende Wirkung entfaltet Leflunomid bzw. Teriflunomid über verschiedene Mechanismen, die im Detail noch nicht analysiert sind.

Der Hauptwirkungs-Mechanismus scheint die reversible Hemmung des Enzyms Dihydroorotat-Dehydrogenase zu sein, das maßgeblich an der *De*-*novo*-Synthese des Pyrimidin-Ribonucelotids Uridinmonophosphat (UMP) beteiligt ist. Die Bildung von stimulierten T-Lymphozyten hängt aber von der *De*-*novo*-Synthese von UMP ab, so dass unter Leflunomid die proliferative Reaktion von Mitogen-stimulierten Lymphozyten ausbleibt.

Zusätzlich werden auch andere Wirkungsmechanismen des Leflunomids diskutiert: die Hemmung der Tyrosinkinase, die Beeinflussung der Cytokin-Bildung, das heißt zum Beispiel die Stimulation der Bildung des immunsuppressiven TGF beta und die Hemmung der Interleukin-2-Synthese. Auch soll Leflunomid die Adhäsion der Leukozyten an das Endothel hemmen. Deshalb bieten sich nicht nur die rheumatoide Arthritis, sondern auch andere Autoimmunerkrankungen als Indikation an. Zudem haben sich in Tierversuchen und *in vitro*-Experimenten Indizien dafür ergeben, dass Leflunomid auch vor Transplantat-Abstoßungen schützt und bei Psoriasis oder Krebs hilft. Leflunomid zeichnet sich in der Regel im Vergleich zu anderen Antirheumatika, wie Sulfasalazin und Methotrexat, durch eine bessere Verträglichkeit aus.

Leflunomid sowie diesen Wirkstoff enthaltende pharmazeutische Zusammensetzungen wurden erstmals in der deutschen Patentanmeldung DE 28 54 439 beschrieben. Das unter der Bezeichnung Arava^{®} vertriebene Leflunomid ist in Form von Tabletten erhältlich, die zur Behandlung der aktiven rheumatoiden Arthritis und aktiven Psoriasis-Arthritis (Arthritis psoriatica) eingesetzt werden.

Aus der europäischen Patentanmeldung EP 0 797 989 ist bekannt, dass die Herstellung von festen Arzneistoffzubereitungen, enthaltend Leflunomid, beispielsweise in Tablettenform, während der Lagerung zur Bildung von 6 - 9% des Abbauproduktes **II** führt (relativ zum anfänglich eingesetzten Leflunomid). Teriflunomid wird dabei während der Lagerung aus Leflunomid gebildet. Obwohl Teriflunomid als aktiver Metabolit des Leflunomids selbst pharmakologisch wirksam ist, ist z.B. aufgrund eines unterschiedlichen Dosis/Wirkung-Verhältnisses in Leflunomid-Zubereitungen enthaltenes Teriflunomid als Verunreinigung zu betrachten.

Arzneistoffzubereitungen, die 6 - 9% eines Abbauproduktes oder einer anderen Verunreinigung enthalten, wie sie gemäß EP 0 797 989 mit herkömmlichen Leflunomid-Zubereitungen erhalten werden, genügen den gegenwärtig gültigen Zulassungsregularien für Arzneistoffe aber in keiner Weise. Die Tabletten müssen vielmehr die heute geltenden Anforderungen an die Stabilität und Reinheit einhalten, wie sie für die Beantragung einer Arzneimittelzulassung erfüllt werden müssen.

Unter Stabilität versteht man nach der international anerkannten APV-Richtlinie (Arbeitsgemeinschaft für pharmazeutische Verfahrenstechnik) "die spezifikationsgerechte Qualität des Arzneimittels bis zum Ende der vom Hersteller festgelegten Laufzeit". Die Qualität des Arzneimittels wird dabei durch den Wirkstoffgehalt und die Reinheit, die sensorisch wahrnehmbaren, physikalisch-chemischen und die mikrobiologischen Eigenschaften bestimmt. Die Anforderungen, wie die entsprechende Stabilitätsprüfung zu erfolgen hat und wie hoch der Anteil an Abbauprodukten bzw. Verunreinigungen in dem zuzulassenden Arzneimittel sein darf, sind beispielsweise in den von der europäischen Zulassungsbehörde (EMEA) herausgegebenen Richtlinien (Guidelines) zur Stabilitätsprüfung (CPMP/QWP/122/02 vom 17. Dezember 2003) und zu Verunreinigungen in neuen Arzneimitteln (CPMP/ ICH/2738/99 vom 20. Februar 2003) festgelegt. Gesamt-Verunreinigungen von mehr als 1% erweisen sich hinsichtlich des arzneimittelrechtlichen Zulassungsverfahrens generell als äußerst problematisch.

Es ist daher erstrebenswert, über pharmazeutische Zusammensetzungen des Leflunomids mit verbesserter Stabilität zu verfügen.

Aufgabe der vorliegenden Erfindung ist es damit, Leflunomid enthaltende pharmazeutische Zusammensetzungen mit verbesserter Stabilität bereitzustellen. Die Aufgabe wird durch die Merkmale des übergeordneten Anspruchs gelöst. Die abhängigen Ansprüche beschreiben bevorzugte Ausführungsformen der vorliegenden Erfindung.

In den der Erfindung zugrundeliegenden Experimenten wurde völlig überraschend gefunden, dass Leflunomid enthaltende feste pharmazeutische Zusammensetzungen mit verbesserter Stabilität erhalten werden, wenn den Zusammensetzungen pharmazeutisch akzeptable, ein- oder mehrbasige organische Säuren zugesetzt werden. Als organische Säuren werden Weinsäure, Äpfelsäure, Fumarsäure, Zitronensäure, Malonsäure, Maleinsäure sowie Methan-, Ethan- und p-Toluolsulfonsäure und Benzolsulfonsäure bevorzugt. Erfindungsgemäß werden insbesondere Weinsäure, Fumarsäure, Zitronensäure, Malonsäure und Maleinsäure bevorzugt.

Auch saure Puffersysteme können erfindungsgemäß zur Stabilisierung der Leflunomid-Zusammensetzungen verwendet werden, wobei in diesem Zusammenhang Verbindungen und Puffersysteme, die in wässriger Lösung einen pH-Wert unterhalb von 7.0 ergeben, als Säure bzw. sauer reagierend bezeichnet werden. Erfindungsgemäß können alle pharmazeutisch akzeptablen, organischen Puffersysteme zum Einsatz kommen; insbesondere werden Citronensäure/Citratpuffer, Weinsäure/Tartratpuffer oder Essigsäure/Acetat-

Puffer bevorzugt. Die Säure oder saure Puffer enthaltenden, erfindungsgemäßen, Leflunomid-Zusammensetzungen zeichnen sich gegenüber solchen Zusammensetzungen, die keinen sauren Zusatz enthalten, durch eine verbesserte Stabilität aus.

Die in den folgenden Vergleichsversuchen verwendeten Tabletten mit und ohne Säurestabilisierung haben folgende Grundzusammensetzungen:

**Tabelle 1: Grund-Rezeptur der Leflunomid-Zusammensetzungen**

| **Komponente** | | **Menge pro Tablette [mg]** | |
|---|---|---|---|
| **intra-granular:** | | | |
| | Leflunomid | 10.00 | 20.00 |
| | Crospovidon (XL) | 3.00 | 6.00 |
| | Laktose Monohydrat (Flowlac 100) bzw. wasserfreie Laktose | 37.00 | 74.00 |
| | Povidon (K-29/32) | 0.36 | 1.00 |
| | Wasser (nach Granulation durch Trocknung entfernt) | | |
| **Summe** | | **50.36** | **101.00** |
| **extra-granular:** | | | |
| | kolloid. Siliziumdioxid | 1.00 | 1.00 |
| | Maisstärke (Purity 826) bzw. vorgekleisterte Stärke | 10.00 | 10.00 |
| | Laktose Monohydrat (Flowlac 100) bzw. wasserfreie Laktose | 68.20 | 18.10 |
| | Povidon (K-29/32) | 1.94 | 1.90 |
| | Crospovidon (XL) | 4.50 | 4.00 |
| | Talkum | 4.00 | 4.00 |
| **Summe** | | **140.00** | **140.00** |
| **Filmbeschichtung** | | | |
| | Opadry II | 4.90 | 4.90 |
| | Wasser (nach Filmbeschichtung durch Trocknung entfernt) | | |
| **Gesamtsumme** | | **144.90** | **144.90** |

Die in den Vergleichsversuchen verwendeten, erfindungsgemäßen Tabletten enthalten zusätzlich intra-granular einen Zusatz einer organischen Säure.

In den erfindungsgemäßen Beispielen 3 bis 11 und 18 wurde Maisstärke und Laktose Monohydrat verwendet, während in den erfindungsgemäßen Beispielen 12 bis 17 und 19 wasserfreie Laktose und vorgekleisterte Stärke zum Einsatz kamen. Alle anderen Komponenten waren in beiden Varianten identisch. Die in den Vergleichsversuchen verwendeten Tabletten des Standes der Technik (Beispiel 2) und die erfindungsgemäßen Formulierungen (Beispiele 3 bis 19) wurden nach folgendem Standard-Herstellungsprozess erhalten.

Zu einer Mischung von Leflunomid, Crospovidon (XL), Laktose Monohydrat (Flowlac 100) bzw. wasserfreier Laktose und (optional) der erfindungsgemäßen Säure wird unter Rühren eine vorgefertigte Lösung von Povidon (K-29/32) in gereinigtem Wasser gegeben und das feuchte Granulat getrocknet; optional kann der gesamte oder ein Teil des erfindungsgemäßen Säurezusatzes auch in der Granulierflüssigkeit gelöst vorliegen. Das trockene Granulat wird mit den extra-granularen Komponenten Siliziumdioxid (kolloid.), Maisstärke (Purity 826) bzw. vorgekleisterte Stärke, Laktose Monohydrat (Flowlac 100) bzw. wasserfreier Laktose und Povidon (K-29/32) vermischt. Zu dieser Mischung werden nacheinander Crospovidon (XL) und Talkum gegeben.

Die so erhaltene Mischung wird zu Tabletten verpresst, die nach den üblichen Methoden filmbeschichtet werden.

Beim Upscaling dieses Verfahrens zu Ansätzen mit technischer Größe sind die zu wählenden Parameter, wie etwa die Scherrate bei der Granulierung oder das Verfahren, die Dauer und die Temperatur der Trocknungen, einer herkömmlichen Anpassung und Optimierung zu unterziehen, insbesondere bietet sich bei den Zusammensetzungen der vorliegenden Erfindung eine Granulierung unter hohem Energieeintrag, also z.B. bei einer großen Scherrate, und eine Fließbetttrocknung zur Trocknung des Granulats an.

Mit den folgenden Beispielen wird das im Stand der Technik existierende Problem der in Leflunomid-Zubereitungen chronisch enthaltenen Teriflunomid-Verunreinigungen sowie die erfindungsgemäße Lösung dieses Problems näher erläutert. Die Beispiele sollen die hiermit beschriebene, erfindungsgemäße Lösung des Problems nicht beschränken.

Die relativen Teriflunomid-Anteile in den Leflunomid-Zusammensetzungen in den folgenden Vergleichsversuchen wurden mittels HPLC bestimmt.

### Beispiel 1:

### Teriflunomidgehalte der reinen Wirkstoff und der kommerziell erhältlichen Produkte

Während der reine Wirkstoff, wie er von Lieferanten bezogen werden kann, nur Teriflunomid-Anteile von 0.01-0.02% (relativ zum Gesamtgewicht des Wirkstoffs) aufweist und somit praktisch frei von dieser Verunreinigung ist, wurden in Testchargen von säurefreien Zusammensetzungen (erhalten nach der Grund-Rezeptur und dem Standard-Herstellungsprozess) bereits unmittel nach ihrer Herstellung zwischen 0.19 und 0.27% Teriflunomid ermittelt. In ähnlicher Weise wurde der Teriflunomid-Anteil in kommerziell erhältlichen, säurefreien Leflunomid-Zubereitungen (ARAVA^{®}-10mg und ARAVA^{®}-20mg) mit 0.23 - 0.46% bestimmt.

### Beispiel 2:

### Lagerstabilität von Leflunomid-Zusammensetzungen des Standes der Technik

Die nach der Grund-Rezeptur und dem Standard-Herstellungsprozess hergestellten, säurefreien Leflunomid-Tabletten wurden teilweise unter Standard- und teilweise unter Stressbedingungen gelagert und der darin enthaltene Teriflunomid-Anteil (relativ zum anfänglich eingesetzten Leflunomid) nach verschiedenen Zeiten bestimmt. Die Ergebnisse wurden in Tab. 2 zusammengefasst.

In Abhängigkeit der Wirkstoffkonzentration und der Lagerbedingungen wurden zum Teil bereits nach 3 Monaten, spätestens jedoch nach 12 Monaten, Teriflunomid-Anteile oberhalb von 1% bestimmt. Die entsprechenden Zusammensetzungen sind spätestens nach diesem Zeitraum als unbrauchbar anzusehen.

**Tabelle 2: Teriflunomid-Anteile (in %, relativ zum anfänglich eingesetzten Leflunomid) in Abhängigkeit der Wirkstoffkonzentration und der Lagerdauer bei Tabletten gemäß dem Stand der Technik.**

| Testdauer | 10 mg Tabletten T=25°C ± 2°C rF=60% ± 5 % | 10 mg Tabletten T=40°C ± 2°C rF=75% ± 5% | 20 mg Tabletten T=25°C ± 2°C rF=60% ± 5% RH | 20 mg Tabletten T=40°C ± 2°C rF=75% ± 5% RH |
|---|---|---|---|---|
| Beginn | 0.20 - 0.27 | 0.20 - 0.27 | 0.19 - 0.20 | 0.19 - 0.20 |
| 3 Monate | 0.37 - 0.39 | 0.81 - 0.95 | 0.43 - 0.49 | 0.79 - 1.04 |
| 6 Monate | 0.49 - 0.52 | 4.39 - 5.11 | 0.63 - 0.72 | |
| 9 Monate | 0.63 - 0.98 | | 0.91 - 1.13 | |
| 12 Monate | 0.82 - 1.10 | | | |
| 25 Monate | 1.32 | | | |

### Beispiele 3-17:

### Stabilität der erfindungsgemäßen Zusammensetzungen

Die nach der Grund-Rezeptur und dem Standard-Herstellungsprozess hergestellten, erfindungsgemäßen, säurehaltigen Leflunomid-Tabletten wurden teilweise unter Standard- und teilweise unter Stressbedingungen in verschiedenen Gefäßen gelagert, der darin enthaltene Teriflunomid-Anteil (relativ zum anfänglich eingesetzten Leflunomid) nach verschiedenen Zeiten bestimmt und mit den entsprechenden Werten von Zusammensetzungen des Standes der Technik (ohne Säurezusatz) verglichen. Die Ergebnisse wurden in den Tabellen 3 bis 5 zusammengefasst.

Die erfindungsgemäßen Zusammensetzungen (Beispiele 3 bis 17 in Tabellen 3 bis 5) zeigen in jedem Fall einen geringeren Anteil an Teriflunomid und somit eine verbesserte Stabilität.

**Tabelle 3: Teriflunomid-Anteile in 10mg Leflunomid Tabletten gemäß dem Stand der Technik (ohne Säurezusatz) und in erfindungsgemäßen 10mg Leflunomid Tabletten (mit Säurezusatz) unter verschiedenen Lagerbedingungen; alle Zusammensetzungen wurden mit Laktose Monohydrat und Maisstärke hergestellt.**

| # | Zusammensetzung | T=40°C rF=75% 5 Tage offen | T= 40°C rF=75% 8 Tage offen | T=25°C rF=75% 14 Tage Flasche | T=25°C rF=75% 30 Tage Flasche | T=40°C rF=75% 30 Tage Flasche | T=40°C rF=75% 14 Tage Blister | T=40° C rF=75 % 30 Tage Blister |
|---|---|---|---|---|---|---|---|---|
| | ohne Säure-Zusatz | 1.24 | 1.40 | 0.10 | 0.17 | 0.74 | 1.94 | 2.77 |
| 3 | Zitronensäure (10 mg) | 0.69 | 0.89 | 0.05 | 0.10 | 0.36 | 0.43 | 0.57 |
| 4 | Weinsäure (10 mg) | 0.37 | 0.42 | 0.05 | 0.12 | 0.28 | 0.18 | 0.20 |
| 5 | Malonsäure (10 mg) | 0.41 | 0.54 | 0.08 | 0.16 | 0.28 | 0.35 | 0.49 |

**Tabelle 4: Teriflunomid-Anteile in 10mg Leflunomid Tabletten gemäß dem Stand der Technik (ohne Säurezusatz) und in erfindungsgemäßen 10mg Leflunomid Tabletten (mit Säurezusatz) unter verschiedenen Lagerbedingungen; alle Zusammensetzungen wurden mit Laktose Monohydrat und Maisstärke hergestellt.**

| # | Zusammensetzung | T=25°C rF=75% 30 Tage offen | T=25°C rF=75% 90 Tage offen | T=25°C rF=75% 180 Tage offen | T=40°C rF=75% 30 Tage HDPE-Flasche | T=40°C rF=75% 90 Tage HDPE-Flasche |
|---|---|---|---|---|---|---|
| | Ohne Säure-Zusatz | 0.17 | 0.14 | 0.18 | 0.74 | 2.43 |
| 6 | Zitronensäure (3 mg) | 0.16 | 0.18 | 0.09 | 0.44 | 2.40 |
| 7 | Zitronensäure (10 mg) | 0.10 | 0.12 | 0.10 | 0.36 | 2.35 |
| 8 | Weinsäure (3 mg) | 0.13 | 0.07 | 0.11 | 0.37 | 2.04 |
| 9 | Weinsäure (10 mg) | 0.12 | 0.08 | 0.09 | 0.28 | 1.24 |
| 10 | Äpfelsäure (3 mg) | 0.19 | 0.14 | 0.17 | 0.63 | 2.23 |
| 11 | Äpfelsäure (10 mg) | 0.16 | 0.05 | 0.12 | 0.58 | 2.18 |

Die in den unter Säurezusatz hergestellten Beispielen 3 bis 11 in den Tabelle 3 und 4 zusammengefassten Daten der Stabilitätsuntersuchungen zeigen damit eindrucksvoll, dass die erfindungsgemäßen Leflunomid enthaltenden festen, oralen pharmazeutischen Zusammensetzungen gegenüber den Tabletten des Standes der Technik eine verbesserte Stabilität besitzen.

Alternativ zu den in den Tabellen 3 und 4 genannten Zusammensetzung wurde in den Beispielen 12-17 (Tabelle 5) wasserfreie Laktose und vorgekleisterte Stärke verwendet. Alle anderen Komponenten und das Verfahren zur Herstellung der Zusammensetzungen blieben zu den Beispielen 3-11 identisch.

**Tabelle 5: Teriflunomid-Anteile in 10mg Leflunomid Tabletten gemäß dem Stand der Technik (ohne Säurezusatz) und in erfindungsgemäßen 10mg Leflunomid Tabletten (mit Säurezusatz) unter verschiedenen Lagerbedingungen; hergestellt mit der alternativen Formulierung unter Verwendung von wasserfreier Laktose und vorgekleisterter Stärke.**

| # | Zusammensetzung | T=25°C rF=75% 90 Tage offen | T=40°C rF=75% 30 Tage HDPE-Flasche | T=40°C rF=75% 90 Tage HDPE-Flasche |
|---|---|---|---|---|
| | ohne Säure-Zusatz | 0.14 | 0.74 | 2.43 |
| 12 | Weinsäure (20 mg) | 0.03 | 0.16 | 1.34 |
| 13 | Weinsäure (30 mg) | 0.05 | 0.41 | 1.40 |
| 14 | Maleinsäure (10 mg) | 0.04 | 0.14 | 0.70 |
| 15 | Maleinsäure (20 mg) | 0.05 | 0.10 | 1.03 |
| 16 | Benzolsulfonsäure (3 mg) | 0.09 | 0.72 | 1.83 |
| 17 | Benzolsulfonsäure (10 mg) | 0.08 | 0.32 | 2.06 |

Auch in den Zusammensetzungen unter Verwendung von wasserfreier Laktose und vorgekleisterter Stärke mit entsprechend reduziertem Wassergehalt zeigt der Wirkstoff Leflunomid in den erfindungsgemäßen Zusammensetzungen eine ausgezeichnete Stabilität gegenüber einer Umlagerung zu seinem Metaboliten Teriflunomid.

Explizite Formulierung besonders vorteilhafter Leflunomid-Zusammensetzungen:

### Beispiel 9:

| **Komponente** | | **Gewicht /mg** |
|---|---|---|
| **intra-granular:** | | |
| | Leflunomid | 10.00 |
| | Crospovidon (XL) | 3.00 |
| | Laktose Monohydrat, Flowlac 100 | 37.00 |
| | Povidon (K-29/32) | 0.36 |
| | Weinsäure | 10.00 |
| | Wasser (nach Granulation durch Trocknung entfernt) | |
| **Summe** | | **60.36** |
| **extra-granular:** | | |
| | kolloid. Siliziumdioxid | 1.00 |
| | Maisstärke, Purity 826 | 10.00 |
| | Laktose Monohydrat, Flowlac 100 | 68.20 |
| | Povidon (K-29/32) | 1.94 |
| | Crospovidon (XL) | 4.50 |
| | Talkum | 4.00 |
| **Summe** | | **150.00** |
| **Filmbeschichtung** | | |
| | Opadry II | 4.90 |
| | Wasser (nach Filmbeschichtung durch Trocknung entfernt) | |
| **Gesamtsumme** | | **154.90** |

### Beispiel 12:

| **Komponente** | | **Gewicht /mg** |
|---|---|---|
| **intra-granular:** | | |
| | Leflunomid | 10.00 |
| | Crospovidon (XL) | 3.00 |
| | Laktose anhydrous | 37.00 |
| | Weinsäure | 10.00 |
| **Summe** | | **60.00** |
| | | |
| **Granulierflüssigkeit:** | | |
| | Povidon (K-29/32) | 0.36 |
| | Weinsäure | 10.00 |
| | Wasser (nach Granulation durch Trocknung entfernt) | |
| **Summe** | | 70.36 |
| | | |
| **extra-granular:** | | |
| | kolloid. Siliziumdioxid | 1.00 |
| | Vorgekl. Stärke, 1500LM | 10.00 |
| | Laktose anhydrous | 48.20 |
| | Povidon. (K-29/32) | 1.94 |
| | Crospovidon (XL) | 4.50 |
| | Talkum | 4.00 |
| **Summe** | | **140.00** |

## Patentansprüche

1. Pharmazeutische Zusammensetzung umfassend Leflunomid und wenigstens eine Säure, **dadurch gekennzeichnet, dass** die pharmazeutische Zusammensetzung in fester Form vorliegt und die Säure eine organische Säure ist, wobei die Säure aus der Gruppe Weinsäure, Äpfelsäure, Malonsäure, Maleinsäure, Zitronensäure, Benzolsulfonsäure, Fumarsäure, Methan-, Ethan- und p-Toluolsulfonsäure ausgewählt ist.

2. Pharmazeutische Zusammensetzung nach Anspruch 1, wobei die Säure Weinsäure ist.

3. Pharmazeutische Zusammensetzung nach einem der Ansprüche 1 und 2, wobei der Gewichtsanteil der Säure relativ zum anfänglich eingesetzten Leflunomid etwa 1:1 bis etwa 3:1 beträgt.

4. Pharmazeutische Zusammensetzung nach einem der Ansprüche 1 bis 3, **dadurch gekennzeichnet, dass** sie in Form von Tabletten oder Kapseln vorliegt.

5. Verfahren zur Herstellung einer pharmazeutischen Zusammensetzung nach einem der Ansprüche 1 bis 4, wobei der Wirkstoff zusammen mit der organischen Säure und optionalen pharmazeutischen Hilfsstoffen tablettiert wird.

6. Verfahren zur Herstellung einer pharmazeutischen Zusammensetzung nach einem der Ansprüche 1 bis 4, wobei der Wirkstoff zusammen mit der organischen Säure und optionalen pharmazeutischen Hilfsstoffen granuliert wird.

7. Verfahren nach Anspruch 6, wobei die Granulation eine Feuchtgranulation ist.

8. Verfahren nach Anspruch 7, wobei als Granulierflüssigkeit eine alkoholische oder wässrige Lösung oder Dispersion verwendet wird.

## Claims

1. Pharmaceutical composition comprising leflunomide and at least one acid, **characterized in that** the pharmaceutical composition is present in solid form and the acid is an organic acid, wherein the acid is selected from the group comprising tartaric acid, malic acid, malonic acid, maleic acid, citric acid, benzene sulfonic acid, fumaric acid, methane-, ethane- and p-toluene sulfonic acid.

2. Pharmaceutical composition according to claim 1, wherein the acid is tartaric acid.

3. Pharmaceutical composition according to any one of claims 1 and 2, wherein the weight amount of the acid in relation to the initially used leflunomide is about 1:1 to about 3:1.

4. Pharmaceutical composition according to any one of claims 1 to 3, **characterized in that** it is present in form of a tablet or capsule.

5. Process for production of a pharmaceutical composition according to any one of claims 1 to 4, wherein the active ingredient is pelletized together with the organic acid and optional pharmaceutical excipients.

6. Process for production of a pharmaceutical composition according to any one of claims 1 to 4, wherein the active ingredient is granulated together with the organic acid and optional pharmaceutical excipients.

7. Process according to claim 6, wherein the granulation is a wet granulation.

8. Process according to claim 7, wherein an alcoholic or aqueous solution or dispersion is used as granulation liquid.

## Revendications

1. Composition pharmaceutique comprenant du léflunomide et au moins un acide, **caractérisé en ce que** la composition pharmaceutique existe en forme solide et que l'acide est un acide organique, l'acide étant choisi du groupe de l'acide tartrique, acide malique, acide malonique, acide maléique, acide citrique, acide benzènesulfonique, acide fumarique, acide méthanesulfonique, acide éthanesulfonique et p-toluènesulfonique.

2. Composition pharmaceutique selon la revendication 1, l'acide étant l'acide tartrique.

3. Composition pharmaceutique selon l'une quelconque des revendications 1 et 2, la part de poids de l'acide relative au léflunomide utilisé initialement étant environ 1:1 jusqu'à environ 3:1.

4. Composition pharmaceutique selon l'une quelconque des revendications 1 à 3, **caractérisé en ce qu'**elle existe en forme de comprimés ou de capsules.

5. Procédé pour la fabrication d'une composition pharmaceutique selon l'une quelconque des revendications 1 à 4, l'agent étant pastillé ensemble avec l'acide organique et des adjuvants pharmaceutiques optionnels.

6. Procédé de fabrication d'une composition pharmaceutique selon l'une quelconque des revendications 1 à 4, l'agent étant granulé ensemble avec l'acide organique et des adjuvants pharmaceutiques optionnels.

7. Procédé selon la revendication 6, la granulation étant une granulation humide.

8. Procédé selon la revendication 7, une solution alcoolique ou aqueux étant utilisée en tant que liquide de granulation.
